# EUROPÄISCHE PATENTSCHRIFT

(11) **EP 2 334 464 B1**
(45) Veröffentlichungstag und Bekanntmachung des Hinweises auf die Patenterteilung: **10.10.2012**
(21) Anmeldenummer: 09778226.2
(22) Anmeldetag: 31.08.2009
(51) Int. Cl.: A61B 18/20, A61F 9/008, B23K 26/00

(54) **MULTIFUNKTIONALES LASERGERÄT**
MULTIFUNCTIONAL LASER DEVICE
DISPOSITIF LASER MULTIFONCTION

(30) Priorität: 29.08.2008 DE 102008044998
(43) Veröffentlichungstag der Anmeldung: 22.06.2011
(73) Patentinhaber: Starmedtec GmbH, 82319 Starnberg (DE)
(72) Erfinder: FALKENSTEIN, Werner, 82319 Starnberg (DE); SCHUBERT, Michael, 82327 Tutzing (DE); WEIDEMANN, Gregor, 81479 München (DE)
(74) Vertreter: Feldkamp, Rainer
(86) Internationale Anmeldenummer: PCT/EP2009/006298
(87) Internationale Veröffentlichungsnummer: WO 2010/022984

(56) Entgegenhaltungen:
- WO-A-98/28104
- US-A1- 2005 225 846

## Beschreibung

Die Erfindung bezieht sich auf ein multifunktionales Lasergerät der im Oberbegriff des Anspruchs 1 genannten Art.

Auf dem Gebiet der Materialbearbeitung allgemein werden in zunehmendem Umfang Laser eingesetzt, die Material abtragen. Hierbei wird typisch mit Lichtimpulsen gearbeitet, die aufgrund der hierbei eingesetzten Festkörper-Laser eine niedrige lmpulswiederholfrequenz, beispielsweise im Bereich von 1 bis 500Hz, jedoch Energien aufweisen, die weit über den Schwellen für einen Abtrag liegen, um einen ausreichend schnellen Abtrag zu erzielen.

Lasergeräte verschiedenster Art werden auch im Bereich der Medizin, beispielsweise auf dem Gebiet der Urologie, der Gastroenterologie, der HNO wie auch anderer medizinischer Disziplinen, eingesetzt, um Obstruktionen bzw. ganz generell Gewebe oder Konkremente aller Art zu entfernen, da sie den Abfluss von Körperflüssigkeiten be- oder verhindern oder anderweitig die Gesundheit oder das Wohlbefinden stören und so zu erheblichen gesundheitlichen Komplikationen führen.

Beispielsweise werden in der Urologie obstruierende Harnsteine in den Nieren, den Harnleitern oder der Harnblase zertrümmert, aber auch obstruierende Gewebeveränderungen wie Neoplasien, Tumore oder Vernarbungen, und gutartige Prostatavergrößerungen entfernt.

Analog treten in der Gastroenterologie Konkremente wie auch Weichgewebeobstruktionen in den galleabführenden Wegen auf, von der Leber, in der die Gallenflüssigkeit erzeugt wird, über die Gallengänge, die Gallenblase bis zum Eintritt des Gallengangs in den Zwölffingerdarm. Ähnliches gilt beim Pankreas mit Pankreassteinen. In der HNO treten Obstruktionen in den Speicheldrüsen und -gängen auf, in der Augenheilkunde in den unteren Tränenwegen vom Auge bis in den Nasenbereich.

Es wurde zunächst versucht, Dauerstrichlaser (Nd:YAG) für die Lithotripsie einzusetzen. Dies führte jedoch zu einer langsamen Aufheizung des Steines, bis auf Temperaturen von über 1000 °C, wobei der Stein zerfiel, jedoch große Schädigungen des umgebenden Gewebes auf Grund der hohen und lang anhaltenden erzeugten Temperatur auftraten.

Auch Diodenlaser im Bereich von 800 - 1000 nm erweisen sich hierfür als ungeeignet.

Es wurde weiterhin festgestellt, dass Nd:YAG-Laser wie auch Diodenlaser im Wellenlängenbereich von 940 nm - 1540 nm im CW-Betrieb beim Abtrag von Gewebe nicht kontrollierbare tiefe Nekrosezonen bei geringem Abtrag setzen, einhergehend mit starker Karbonisation. Während erstere vermieden werden sollten, da hierbei benachbarte Organe in Mitleidenschaft gezogen werden können, sind letztere als solche nicht unbedingt schädlich für die Gesundheit des Patienten. In der Energiebilanz ist dies jedoch ein Prozess mit hohem Energieverbrauch und damit im Hinblick auf einen effizienten Abtragungsprozess nachteilig. Entsprechend wird der Nd:YAG-Laser in der Regel nicht mehr eingesetzt.

Stattdessen werden im medizinischen Bereich derzeit hauptsächlich gepulste Holmiumlaser mit einer Wellenlänge von 2080 nm eingesetzt, um harte Konkremente zu zertrümmern oder auch Hart- und Weichgewebe zu verdampfen. Hierzu werden Impulsenergien von 200 mJ bis zu 4200 mJ eingesetzt, die Wiederholraten sind im Bereich von 3 Hz bis zu 50 Hz, die Impulsdauern liegen im Bereich von 150 µs - 700 µs.

Die Impulsspitzenleistung eines einzelnen Laserimpulses eines typischen Holmiumlasers beträgt bis zu 15 kW. Diese wird neben der Wellenlänge als bestimmend für die Wechselwirkung des Laserlichtes mit dem Stein bzw. dem Gewebe angesehen. Die Wirkungsweise ist, gleich ob Stein oder Gewebe, weitgehend vergleichbar: das im Stein/Gewebe befindliche Wasser dient als hauptsächlicher Absorber der Wellenlänge des Holmiumlasers mit einem hohen Absorptionskoeffizienten von 28 cm-¹, es wird bei Applikationen der verwendeten Laserimpulse mit den genannten Impulsdauern schlagartig auf eine Temperatur von mehreren 100 °C gebracht, die über dem regulären Siedepunkt von Wasser (100°C) liegt (überhitztes Wasser). Dieses Wasser verwandelt sich in hoch komprimierten Wasserdampf, der, da noch auf das kleine Bestrahlungsvolumen beschränkt, extrem schnell expandiert und so Gewebe- wie Stein partikel mit sich reisst. Dieser Abtragprozess ist deshalb so interessant, da nur ein Bruchteil, Abschätzungen ergeben ca. 30% des bestrahlten Volumens mit dem dafür notwendigen Energieaufwand in Dampf umgewandelt werden muss, der Rest wird durch den rasch expandierenden Wasserdampf mechanisch mitgerissen. Dieser Prozess funktioniert nur dann optimal, wenn die Bedingungen des "Heat confinement" gegeben sind, das heißt die Impulsdauer der applizierten Strahlung deutlich kürzer ist, als die für das Material charakteristische Zeit für die Wärmeleitung (thermische Relaxationszeit). Ist diese Bedingung erfüllt, dient im Wesentlichen die gesamte applizierte Energie dem Abtrag, während nur ein unwesentlicher Bruchteil innerhalb dieser Zeit in benachbarte Gewebeareaie abfließen kann. Der Abtrag erfolgt entsprechend weitgehend athermisch, der erzeugte Krater im Gewebe wird nur von einem dünnen Saum von koaguliertem Gewebe umgeben, der zwar häufig ausreicht, kleinere Blutgefäße zu versiegeln, aber dennoch nicht zu einer völlig blutungsfreien Behandlung führt. Weiterhin ist die Größe der erzeugten Krater abhängig von der Impulsenergie, je größer diese ist, umso größer auch Kratertiefe und -durchmesser. Es können so einzelne Krater aneinander gereiht werden, ähnlich den Stichen einer Nähmaschine, aber kein glatter, feiner Schnitt, wie z.B. beim Einsatz eines Skalpells erreicht werden.

Damit sind sind Holmiumlaser dazu geeignet, sowohl Steine zu zertrümmern als auch Gewebe abzutragen bzw. dieses zu schneiden, dieses allerdings nur in vergleichsweise grober Weise.

Während Holmiumlaser für die reine Steinbehandlung mit Leistungen von 10-20 W, Energien von 0,2-2J und Wiederholraten von 3-10 Hz als Desktopgeräte gebaut werden können, mit Abmessungen von ca. 50x50x30 cm³ und einem Gewicht von 30-40 kg, sind für höhere Leistungsklassen von 60-100 W mit Energien von 0,2-4,2J und Wiederholraten von bis zu 50 Hz große Standgeräte erforderlich, die Abmessungen von 50x110x80 cm³ aufweisen, und bis zu 160 kg schwer sind. Diese großen, schweren und auch teuren Geräte sind erforderlich, um die für einen ausreichenden Gewebeabtrag oft für erforderlich gehaltenen hohen Wiederholraten von 30-50 Hz zu erzeugen. Typisch werden mehrere Laserstrahlquellen zeitlich versetzt jeweils mit geringerer Frequenz betrieben, um so die erforderliche externe hohe Wiederholrate zu erzeugen, z.B. 4 Laserstrahlquellen mit jeweils 10 Hz und 20 W zur Erzeugung von 40 Hz, 80 W.

Derzeitige Holmiumlaser werden daher gegenwärtig vorzugsweise für die Lithotripsie verwender und weisen Fragmentationsraten von 0,2 mg/Impuls bei einer Energie von 1000 mJ auf. Dies führt zu Fragmentationsraten von 120 mg/Minute bei 1000 mJ/10 Hz und hochgerechnet 240 mg/min bei 2000 mJ/10 Hz. An diesen Fragmentationsraten müssen sich zukünftige Lithotripsiegeräte messen lassen.

Weiterhin werden neben den Holmiumlasern Dauerstrich- (CW-) Laser im Wellenlängenbereich von 1,8 - 2,1 µm eingesetzt, die Gewebe fein und präzise schneiden und auch verdampfen, die aber in der Regel Steine nicht zertrümmern können, so dass ihr Einsatzbereich beschränkt ist. Typische derzeitige 2 µm CW-Laser sind:
- Dioden gepumpte Thuliumlaser mit einer festen Wellenlänge von 2010 nm
- Dioden gepumpte Faserlaser mit festen Wellenlängen im Bereich von 1900 - 2000 nm.
- Laserdioden im Bereich von 1800 - 2100 nm auf Basis von (AlGaln)(Gasb).

Gegenwärtig im Einsatz befindliche Thulium- und Faserlaser können zwar getaktet werden, hierdurch ändert sich aber die Spitzenleistung nicht: ein z.B. bei 100 W betriebener Laser kann extern moduliert werden, z.B. eine Impulsdauer von 10 ms eingestellt werden, in gleichem Maß reduziert sich aber auch die Energie auf 1 J, so dass sich wiederum eine Impulsspitzenleistung von 100 W ergibt, bei einer Wiederholrate von z.B. 10 Hz die abgegebene Leistung von 100 W auf 10 W sinkt.

Für einen präzisen chirurgischen Effekt mit kontrollierbaren geringen bzw. in ihrer Größe gewünschten Nebeneffekten (z.B. Koagulationszone) müssen die Parameter der Energiequelle (hier: Lasergerät) geeignet ausgewählt werden. Um überhaupt einen chirurgischen Effekt zu erzielen, müssen in dem zu bearbeitenden Material Absorber vorliegen, die die Wellenlänge des applizierten Lichtes absorbieren und so den gewünschten Effekt erzeugen. Im menschlichen Körper liegen unterschiedliche Absorber vor, deren wichtigste Wasser, Blut und Melanin sind.

Entsprechend der Höhe der Absorption bei der eingestrahlten Laserwellenlänge und der Konzentration des Absorbers ergeben sich Eindringtiefe und (chirurgische) Effekte. Besonders geeignet erscheinen Absorptionsspitzen (z.B. Blut bei 540 nm bzw. 580 nm) und die verschiedenen Wasserabsorptionsspitzen im mittleren Infrarotbereich (1,5 / 2 / 3 µm), um eine möglichst selektive Wirkung unter weitestgehender Schonung benachbarter, darüber- oder darunterliegender Gewebe zu erzeugen. Die Absorptionskoeffizienten und damit die Eindringtiefen variieren hierbei erheblich. Die Eindringtiefe sollte hierbei so gewählt werden, dass sie der Größe der Zielstruktur angepasst ist. Soll z.B. die Epidermis mit einer Dicke von ca. 100 µm abgetragen werden, erscheint der Erbiumlaser mit einer Wellenlänge von 2,94 µm am besten geeignet, da er das Gewebe in Schichten von einigen 10 µm abzutragen gestattet und somit in mehreren Durchgängen die Epidermis gezielt abgetragen werden kann.

Demgegenüber eignet sich die Absorptionsspitze bei 2 µm mit einer Eindringtiefe der Laserstrahlung von etwa 0,1 bis 0,5 mm (entspricht einem Absorptionskoeffizienten von 100 cm-¹ bis 20 cm-¹) gut für chirurgische Anwendungen allgemeiner Art, bei der die Größe der Zielstrukturen im Bereich von einigen Millimetern bzw. bis zu Zentimetern (beim Schneiden) liegt.

Häufig wird der Wellenlängenbereich von 1800-2100 nm einfach nur als der Bereich eines der Absorptionspeaks von Wasser angesehen, ohne auf die detaillierte Struktur des Absorptionskoeffizienten von Wasser und dessen Auswirkungen auf die Licht-Material-Wechselwirkung zu achten. Die Absorptionsspitze weist von 1,92-1,94 µm hohe Werte von 120-130 cm⁻¹ auf. Der gesamte Bereich erscheint gleichermaßen geeignet zu sein. Ein hoher Absorptionskoeffizient bedeutet eine geringe Abtragsschwelle und damit einen effizienten Abtrag bei gegebener Energie bzw. Leistung.

Zum anderen bedeuten Absorptionspeaks, dass der Absorptionskoeffizient zu beiden Seiten der Spitze mehr oder weniger stark abfällt, und sich damit die Absorptionseigenschaften, die damit verbundene Eindringtiefe und sich so die Licht-Gewebe-Wechselwirkung teilweise massiv ändern.

Bisheriger Stand der Technik ist es, zwei Laser unterschiedlicher Wellenlänge (1,06 µm und 1,32 µm oder 980 nm und 2,0 µm) zu verwenden, um unterschiedliche Licht-Gewebe-Wechselwirkungen zu erzeugen.

Aus der EP-B-1079897 ist es weiterhin bekannt, bei unveränderter Wellenlänge (z.B. 2,94 µm) durch Modulation der Emission eines Er-Lasers in gewissen Grenzen von rein abtragenden Impulsen auf Impulse mit einer gewissen begrenzten oberflächlichen thermischen Wirkung (Koagulationszonen 100 µm) zu wechseln. Diese Koagulationszone ist z.B. ausreichend, um feinste kapilläre Blutungen der oberen Dermis zu verschließen, aber nicht, um größere Blutgefäße sicher zu koagulieren. Die Änderung der Licht-Gewebe-Wechselwirkung ist hierbei also recht begrenzt.

Für einen effizienten Abtrag ist es aber auch erforderlich, dass die applizierten Energie weitestgehend für den Abtrag zur Verfügung steht und nicht durch konkurrierende Prozesse, wie Wärmeleitung und Strukturänderung verloren geht. Um die Bedingungen des "thermal confinement" - "thermischen Einschlusses" zu erfüllen, muss die Impulsdauer deutlich kürzer sein als die thermische Relaxationszeit, die bei biologischem Material im Wesentlichen durch den Wasserabsorptionskoeffizienten gegeben ist. Für eine Impulsdauer, die einen Faktor >2 unter dieser rechnerischen Relaxationszeit liegt, ist zum Ende des Laserimpulses die gesamte applizierte Energie im bestrahlten Volumen konzentriert, während bei Impulsdauern, die einen Faktor >2 über dieser rechnerischen Größe liegen, sich die absorbierte Energie zum Ende des Laserimpulses über ein größeres Volumen verteilt und sich so die Spitzentemperatur im bestrahlten Volumen beträchtlich reduziert hat.

Es ist zu errechnen, dass für den Bereich von 1920 nm-1940 nm von Wasser die thermische Relaxationszeit etwa 8 ms beträgt und demzufolge die Bedingungen des thermischen Einschlusses bei Impulsdauern unter 4 ms gegeben sind. Dies bedeutet konkret, dass für einen effizienten Abtrag, gleichgültig ob Stein oder Gewebe, Impulsdauern von <4 ms zu bevorzugen sind.

Da in der Regel nicht nur das im Gewebe befindliche Wasser das eingestrahlte Licht absorbiert, sondern auch das Gewebe selbst, ergibt sich eine weitere Grenze: die Absorption der Laserimpulse führt unmittelbar nach dem eigentlichen Absorptionsprozess zu mechanischen Ausdehnungseffekten, die die eingestrahlte Energie abführen, noch bevor die Effekte der Wärmeleitung einsetzen. Bei diesen deutlich kürzeren Einstrahldauern kann es zu sogenannten Stresswellen führen, und gelingt es, den Effekt der Stresswellen auf das bestrahlte Volumen zu begrenzen, führt dies zu maximalen Zug- und Druckwellen, die den Gewebeverbund mechanisch aufreißen. Dies kann wiederum zu einer nochmaligen Erhöhung der Abtragseffizienz von Gewebe führen. Typisch sind, abhängig von der Gewebestruktur, Impulsdauern von fs bis ns erforderlich, um diese Bedingung zu erfüllen.

Da Gewebe typischerweise aus unterschiedlich großen Strukturen aufgebaut sind (Ausnahme: Cornea), können nur generelle Aussagen getroffen werden, in der Art, dass eine Verkürzung der Impulsdauern vom Millisekundenbereich über Mikrosekunden bis in den Nano- und Pikosekundenbereich Verbesserungen in der Ablationseffizienz erwarten lassen, diese ist für jeden Gewebetyp und Wellenlänge in Abhängigkeit von der Impulsdauer getrennt in vivo zu ermitteln.

Aufgrund der Weiterentwicklung von Diodenlasern in Richtung auf eine höhere Leistung und einen höheren Gesamt-Wirkungsgrad werden zunehmend auch Diodenlaser eingesetzt, da sie als Tischgeräte konzipiert werden können. Wesentlich ist hierbei zusätzlich, dass nicht nur die nominale, vom Lasergerät abgegebene Leistung relevant ist, sondern die im Gewebe tatsächlich zur Verfügung stehende Leistung betrachtet werden muss. Diese ergibt sich aus der Geräteleistung nach Abzug von Oberflächenreflektion und Rückstreuung. Letztere beträgt ca. 55% bei 1064 nm, 25% bei 940-980 nm, aber nur 5% bei 2 µm Wellenlänge, was wiederum bedeutet, dass der mittlere Infrarotbereich von 1,4 bis 3,5 µm zu bevorzugen ist.

Derzeit stehen Laserdioden auf der Basis von (A1Gain)(GaSb) oder (A1Galn)(inP) zur Verfügung, die teilweise direkt im Wellenlängenbereich von 1800-2300 nm abstrahlen. Diese Laserdioden werden als reine Dauerstrichlaser mit Leistungen von bis zu 18 W angeboten. Höhere Leistungen sind erst in einigen Jahren zu erwarten.

Aus der DE 199 12 992 ist ein Verfahren zur punktuellen oder flächigen Lichtbestrahlung unter Verwendung von Laserdioden bekannt, bei dem die Impulsleistung und/oder die Impulsdauer und/oder die Wiederholfrequenz bei weitgehend konstanter mittlerer Leistung vom Benutzer oder automatisch in Abhängigkeit von der angestrebten Wirkung geändert werden können, wobei eine Anzahl von Laserdioden gleichzeitig oder alternierend verwendet wird. Dennoch dürfte die erreichbare Abtragrate begrenzt sein. Weiterhin wird hierbei eine einzige feste Wellenlänge verwendet, die durch die Art der Laserdiode oder Laserdioden festgelegt ist.

Aus dem Vorstehenden ist zu erkennen, dass bisher für die jeweiligen Anwendungszwecke unterschiedliche oder aufwändige Lasergeräte erforderlich waren, die einen retativ großen Raum- und Leistungsbedarf haben und kostspielig sind.

Der Erfindung liegt die Aufgabe zu Grunde, ein multifunktionales Lasergerät zu schaffen, das bei einfachem und raumsparenden Aufbau und geringem Leistungsbedarf einen weiten Anwendungsbereich ergibt.

Diese Aufgabe wird ausgehend von einem multifunktionalen Lasergerät zur Bearbeitung von biologischem Material, das eine starke Veränderung der Absorptionseigenschaften über den Betriebs-Wellenlängenbereich aufweist, mit mindestens einer aus einer steuerbaren Stromversorgung gespeisten Laserdiode, wobei die Stromversorgung mit einer Steuereinrichtung zur Ansteuerung der Laserdioden mit veränderbarem Strom und/oder veränderbarer Impulsdauer und/oder Impulswiederholfrequenz versehen ist, erfindungsgemäß dadurch gelöst, dass die die Steuereinrichtung für eine schnelle Änderung der Laserlicht-Wellenlänge von einer ersten Wellenlänge auf eine zweite Wellenlänge zur Anpassung an den vorgesehenen Anwendungszweck über eine Zeit im Bereich von weniger als ein Sekunde ausgebildet ist und dass die Steuereinrichtung zur Steuerung des Stromes an die mindestens eine Laserdiode derart ausgebildet ist, dass sich die schnelle Änderung der Wellenlänge über einen Betriebs-Wellenlängenbereich ergibt, der die Wellenlänge zumindest eines Bereichs mit einer wesentlichen Änderung des Absorptionskoeffizienten (Absorptionsspitze, starker Anstieg oder Abfall der Absorption) des Materials einschließt.

Gemäß einer weiteren Ausgestaltung der Erfindung ist vorgesehen dass die Steuereinrichtung zur Änderung der Wellenlänge durch Änderung des Dauerstrich-Laserdioden-Stromes ausgebildet ist.

Gemäß einer weiteren Ausgestaltung der Erfindung ist vorgesehen dass die Steuereinrichtung zur Änderung der Wellenlänge durch Änderung des Verhältnisses von Impulsdauer zur Impulswiederholzeit des Laserdioden-Stromes ausgebildet ist.

Die Steuereinrichtung kann hierbei zur Änderung des Verhältnisses von Impulsdauer zur Impulswiederholzeit des Laserdioden-Stromes von Impuls zu Impuls oder von Impulsgruppe zu Impulsgruppe ausgebildet sein.

Die Steuereinrichtung kann eine erste Betriebsart aufweisen, in der die Impulsdauer während einer Impulswiederholzeit größer als die Impulsdauer für einen Quasi-Dauerstrich-Betrieb ist.

Die Impulsdauer in dieser ersten Betriebsart ist vorzugsweise größer als 1µs und weiter bevorzugt größer als 10µs.

Der Diodenlaser-Strom ist vorzugsweise während der Impulse in einer Impulswiederholzeit bei Impulsdauern von 1-100 µs um den Faktor 5 bis 50 gegenüber dem Nennstrom des Diodenlasers vergrößert.

Die Steuereinrichtung kann weiterhin eine zweite Betriebsart aufweisen, in der die Impulsdauer während einer Impulswiederholzeit kleiner als die Impulsdauer für einen Quasi-Dauerstrich-Betrieb ist, wobei die Impulsdauer in der zweiten Betriebsart im Piko- bis Nanosekunden-Bereich liegen kann.

In dieser zweiten Betriebsart kann der Diodenlaser-Strom während der Impulse in einer Impulswiederholzeit bei Impulsdauern von 10-1000 ns um den Faktor 10 bis 100 gegenüber dem Nennstrom des Diodenlasers vergrößert sein, während er bei Impulsdauern von 10ps-10 ns um den Faktor 50 bis 1000 gegenüber dem Nennstrom des Diodenlasers vergrößert sein kann.

Gemäß einer vorteilhaften Ausgestaltung der Erfindung können zumindest zwei Diodenlaser vorgesehen sein, deren Ausgangssignal einer Kombinationseinrichtung zugeführt wird.

Gemäß einer weiteren vorteilhaften Ausgestaltung der Erfindung kann die Steuereinrichtung zur Veränderung des Stromes, des Verhältnisses von Impulsdauer zur Impulswiederholzeit und der Wellenlänge in Abhängigkeit von dem Ausgangssignal eines Detektionssystems ausgebildet sein, das zur Erkennung von Konkrementen, Blutgefäßen, Nerven, Grenzschichten (Tumor, Prostatakapsel o.a.) über eine Erkennung der Autofluoreszenz von Gewebe, Detektion einer Fluoreszenz nach Anregung über eine zweite Lichtquelle und/oder in Kombination mit ORT ausgebildet ist.

Die Steuereinrichtung kann in vorteilhafter Weise zur Einstellung der Wellenlänge des Ausgangslichts des oder der Diodenlaser auf die Wellenlänge im Bereich der Absorptionsspitze des zu bearbeitenden Materials bei dessen jeweiliger Temperatur ausgebildet sein.

Bevorzugt ist hierbei die Steuereinrichtung zur Einstellung der Wellenlänge des Ausgangslichts des oder der Diodenlaser auf eine Wellenlänge im Bereich der Absorptionsspitze des zu bearbeitenden Materials ausgebildet, bei der die Absorptionsspitze eine geringe Abhängigkeit von der Temperatur des zu bearbeitenden Materials aufweist.

Diese Absorptionsspitze kann die Absorptionsspitze von Wasser sein.

Durch die erfindungsgemäße Ausgestaltung des multifunktionalen Lasergerätes ist ein weiter Anwendungsbereich bei geringer Leistungsaufnahme und Raum und Gewicht sparendem Aufbau möglich.

Damit kann ein kostengünstiges Gerät mit kleinen Abmessungen als Tischgerät mit optimierten Eigenschaften nicht nur für das präzise Schneiden (CW-Betrieb) sondern auch für die Entfernung bzw. Zerkleinerung von Obstruktionen aller Art - Steine, verschiedene Gewebearten, mit kleinen Abmessungen und geringem Gewicht, günstigen Herstellkosten, intraoperativ schnell veränderlicher Wellenlänge und/oder Impulsdauer geschaffen werden.

Eine Lithotripsie kann weiterhin mit geringen Energien, kurzen Impulsdauern, aber hohen Repetitionsraten ohne zusätzlichen Aufwand für einen Güteschalter durchgeführt werden, ohne dass die Gefahr eines Festklebens einer Faserspitze am Gewebe oder einer extremen Überhitzung und eines Abbrennens der Faserspitze besteht, da bei gepulstem/getaktetem Betrieb die Pausen lang genug gewählt werden können, um diese hohen Temperaturen an der Faserspitze sicher zu vermeiden.

Während Festkörperlaser im Bereich des nahen und mittleren infrarotbereichs wie der Neodym, der Holmium- oder der Thuliumlaser physikbedingt, aber auch Faserlaser auf Grund des die Wellenlänge bestimmenden Bragg-Gitters am Faserende in ihrer Wellenlänge im Wesentlichen festgelegt sind, weisen Diodenlaser typischerweise eine gewisse, vom jeweiligen System abhängige Temperaturverschiebung der Wellenlänge auf. In der Regel wird versucht, diese zu minimieren, sei es durch eine spezifische Auslegung der Abfolge von Schichten im Aufbau des Diodensystems, sei es durch starke thermische Anbindung an (Mikrokanal-) Kühler und deren aktive oder passive Kühlung. Natürlich kann damit über z.B. eine externe Kühlmitteltemperatur bzw. das Maß des Luftdurchsatzes bei Luftkühlung auch in gewissen Grenzen die Emissionswellenlänge auf einen gewünschten Sollwert eingestellt werden. Dieser Prozess ist aber langsam (typischerweise mehr als eine Minute).

Ein Diodensystem mit einer vergleichsweise größeren Temperaturabhängigkeit kann demgegenüber über einen weiteren Wellenlängenbereich eingestellt werden und so die Lichtquelle über einen größeren Bereich einer Absorptionskurve verschoben werden, so dass die sonst unerwünschte Temperaturabhängigkeit in vorteilhafter Weise zur Erzielung der gewünschten Wirkung ausgenutzt werden kann.

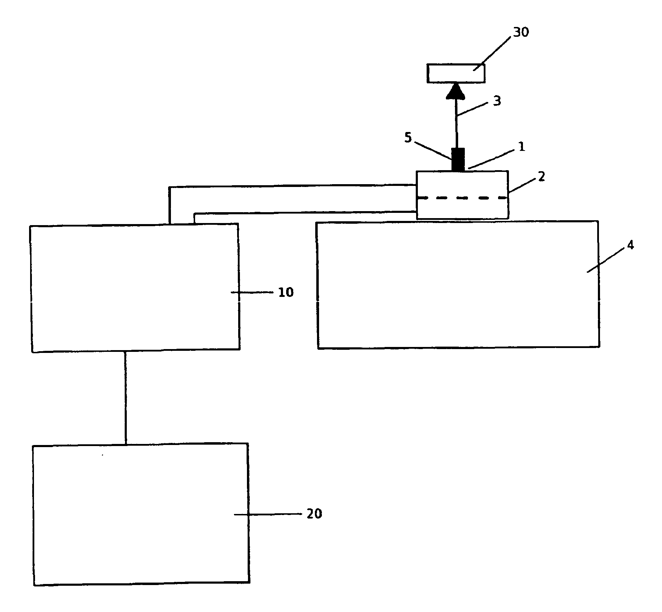

Die Erfindung wird weiter anhand einer in der Zeichnung gezeigten schematischen Darstellung eines Lasergerätes näher erläutert.

In der Zeichnung ist eine Ausführungsform eines Lasergerätes gezeigt, das zumindest eine auf einem Kühlelement 4 in wärmeleitender Verbindung mit diesem angeordnete Laserdiode 1 mit einer Grenzschicht 2 aufweist.

Das Kühlelement 4 kann eine steuerbare Kühlleistung aufweisen.

Das Ausgangslicht der zumindest einen Laserdiode 1 wird über ein Auskoppelelement 5 in eine Lichtleitfaser 3 eingekoppelt, deren freies Ende auf ein zu bearbeitendes Material 30 gerichtet oder mit diesem in Kontakt gebracht wird.

Die Laserdiode 1 wird aus einer Stromversorgung 20 über eine Steuereinrichtung 10 mit einem Strom gespeist, der durch die Steuereinrichtung in vielfältiger steuer- und programmierbarer Weise änderbar ist.

Eine schnelle Veränderung der Wellenlänge kann sehr einfach bewerkstelligt werden, wenn berücksichtigt wird, dass die Lumineszenzwellenlänge von derartigen Laserdiodensystemen, das heißt die Wellenlänge bei geringer Leistung und die Arbeitswellenlänge auf Grund der Temperaturabhängigkeit des jeweiligen Materialsystems deutlich unterschieden sind. So differiert die Lumineszenzwellenlänge von (A1Galn)(GaSb)-Laserdioden im Wellenlängenbereich von 2000 nm um bis zu 55 nm von der Arbeitswellenlänge, die bei Maximalstrom und damit Maximalleistung definiert ist. So ergaben eigene Messungen an einem Diodenmodul mit nominal 13 W Maximalleistung folgende Werte:
70 A 13 W / 1940 nm
30 A / 5 W / 1905 nm
15 A / 2,3 W / 1885 nm

Bei einer Wellenlänge von 1920 nm bei Maximalleistung ist demzufolge eine Wellenlänge von 1865 nm für eine Leistung von 2,3 W zu erwarten. Der Absorptionskoeffizient von Wasser ändert sich hierbei von 128 cm-¹ auf etwa 17 cm⁻¹.

Die Wellenlänge der Diode 1 folgt ohne wesentliche Zeitverzögerung der Temperatur der Grenzschicht für den Laserübergang, so dass über eine schnelle Änderung des Diodenstroms die Wellenlänge gleichermaßen schnell verändert werden kann, allerdings unter Veränderung der abgegebenen optischen Leistung. Dies kann jedoch durchaus erwünscht sein, wenn z.B. zunächst mit einer Wellenlänge gearbeitet wird, bei der eine hohe Materialabsorption vorliegt, dann aber bei Treffen auf ein anderes Material 30 mit anderen Eigenschaften mit geänderter Wellenlänge und vielleicht sogar verminderter Leistung gearbeitet werden soll.

Die Lumineszenzwellenlänge liegt aber nicht nur bei ganz geringen Leistungen vor, sondern auch bei sehr kurzen Impulsdauern deutlich unter ca. 1 µs, ein Wert oberhalb dem für die Laserdiode im Wesentlichen der CW-Betriebsbereich vorliegt. Soll jedoch z.B. die abgegebene optische Leistung konstant gehalten und nur die Wellenlänge geändert werden, kann dies in der Form geschehen, dass nicht mit maximaler Leistung gearbeitet wird, aber mit Impulsen unterschiedlicher Impulsdauer und Wiederholfrequenz: z.B. für Wellenlänge 1 mit Impulsen im Quasi-CW-Betrieb mit Impulsen >>1 µs, z.B. 20 µs, aber geringer Wiederholfrequenz und für die Wellenlänge 2 mit kurzen Impulsen <<1 µs z.B. 200 ns, aber höherer Wiederholfrequenz.

Damit kann nun ein Lasersystem in einfacher und gegebenenfalls auch schneller Weise (<1 sec) in diesem Bereich nicht nur hinsichtlich seiner abgegebenen Energie, sondern auch hinsichtlich seiner Wellenlänge verändert werden, so dass die Wechselwirkung Licht-Material (Wasser/Gewebe) in noch größerem Maße verändert werden und so besser an den vorgesehenen Anwendungszweck angepasst werden kann. So kann von hoher Absorption und damit geringerer Eindringtiefe gut für nebenwirkungsarmes präzises Schneiden von Gewebe, schnell auf verringerte Absorption mit größerer Eindringtiefe und damit verstärkten thermischen Wirkungen z.B. zur verbesserten Blutstillung größerer Gefäße gewechselt werden.

Die in der Literatur angegebenen Werte für den Absorptionskoeffizienten werden in der Regel für Zimmertemperatur bestimmt (20 °C). Es wurde festgestellt, dass der Absorptionskoeffizient von Wasser Temperatur abhängig ist und die Absorptionskurve bei höheren Temperaturen leicht ansteigt und sich zu kürzeren Wellenlängen hin verschiebt. Da der Abtrag von Stein oder Gewebe in der Regel bei Temperaturen um den Siedepunkt erfolgt, ist es sehr viel realistischer, den Absorptionskoeffizienten bei 80 °C als Grundlage für die Optimierung von Gerätespezifikationen heranzuziehen. Will man sich die Vorteile eines möglichst hohen Absorptionskoeffizienten zu Nutze machen, ergibt sich hieraus, dass unter Berücksichtigung des Temperatur abhängigen Absorptionskoeffizienten von Wasser eine Wellenlänge von 1,92 µm anderen Wellenlängen vorzuziehen ist, da dieser bei Temperaturerhöhung von 20 °C sogar leicht von 128 1 /cm auf 145 1 /cm anwächst, während z.B. 1,94 µm - bei 20 °C weitgehend gleich zu 1,92 µm, demgegenüber deutlich von 128 1 /cm auf 120 1 /cm abfällt.

Derzeit stehen Laserdioden auf der Basis von (AlGaIn)(GaSb) oder (AlGaIn)(InP) zur Verfügung, die teilweise direkt im Wellenlängenbereich 1800-2300 nm abstrahlen. Diese Laserdioden werden als reine Dauerstrichlaser mit Leistungen von bis zu 18 W angeboten.

Diese Diodenlaser sind derzeit in Abhängigkeit vom Wellenlängenbereich in unterschiedlichen Leistungsvarianten erhältlich:
940-980 nm: Leistungen bis 150 W / 200 W
1800-2200 nm: Leistungen bis zu 18 W

Es ist bekannt, dass Diodenlaser mit geeigneten Ansteuerungen ohne zusätzliche externe Güteschalter gepulst betrieben werden können. Da die Diodenfacetten in der Regel nur bestimmte Maximalleistungen aushalten, ergibt sich hier nur eine mäßige typische Impulsüberhöhung von 20-50% (CW: 120 W, getaktet im Bereich von Impulsdauern von mehr als 10 ms: 150 W).

Es wurde jedoch erfindungsgemäß festgestellt, dass beispielsweise Laserdioden auf der Basis (AlGaIn)(GaSb) im Impulsdauerbereich von einigen ps über 100 ns bis ca. 100 µs um erhebliche Faktoren (derzeit Faktor 50 bei 50 ps bis Faktor 10 bei 10 µs) überpulst werden können, ohne dass es zu einer Schädigung der Facetten bzw. zu einem irreversiblen thermischen roll-over kommt, das heißt gegenüber der CW-Leistung steigt die Impulsspitzenleistung um einen entsprechenden Faktor an. Damit können Leistungseinbußen, wie sie bei der Taktung von z.B. Dioden gepumpten Faserlasern oder Dioden gepumpten Festkörperlasern zwangsläufig sind, zumindest teilweise kompensiert werden.

Bei geeigneter Optimierung für den Betrieb mit Überpulsung gegenüber dem CW-Betrieb sind weitere Verbesserungen im Hinblick auf höhere Leistung der Einzelemitter und das Maß der Überpulsbarkeit zu erwarten.

Die Impulsdauer kann so leicht in einen Bereich verlegt werden, bei dem die Bedingungen des heat-confinement oder sogar des stress-confinement vorliegen, und ein entsprechender hocheffizienter Abtrag erzielt werden.

Erfindungsgemäß wird aufbauend auf dem höheren Absorptionskoeffizienten und damit geringeren Abtragsschwellen mit vergleichsweise geringen Impulsenergien, aber mit entsprechend höheren Frequenzen, ein suffizienter Abtrag auch an harten Steinen erzielt, indem die Wellenlänge auf eine Absorptionsspitze des jeweiligen Materials eingestellt und eine entsprechend hohe Wiederholfrequenz gewählt wird.

Unter Einsatz eines CW-Faserlasers durchgeführte Untersuchungen ergeben Folgendes:
Abtragsschwelle bei 1,92 µm und 230 µm Faser: 0,15 mJ/2000 Hz; 0,23 mJ/1000 Hz; 0,32 mJ/500 Hz;
Abtragsschwelle bei 1,92 µm und 365 µm Faser:0,25 mJ/2000 Hz; 0,51 mJ/1000 Hz; 1,02 mJ/500 Hz;
(Einbeziehung der Fläche: Erhöhung um Faktor 2,5; Experiment: im Mittel 2,357).

Hieraus kann geschlossen werden, dass die Abtragsschwelle im Bereich von 0,2-1,3 mJ zu liegen kommt.

In praxi wird zur Erzielung eines guten Abtrags deutlich über der Ablationsschwelle gearbeitet. Verwendet man ein 50 W-Gerät mit einem Duty cycle von 50%, also effektiv 25 W und 500 Hz, so ergibt sich eine Einzelimpulsenergie von 50 mJ mit einer Impulsdauer von 1 ms (Faktor 250-400 über der Abtragsschwelle).

Bei 500 Hz/25 W konnte so an einem natürlichen Kalziumoxalatmonohydrat-Stein (sehr harter Stein, eine Fragmentationsrate von 52 mg/min und damit eine Verdoppelung der Abtragseffizienz gegenüber Literaturwerten erzielt werden.

Eine Vergleichsmessung mit einem Dioden gepumpten Thulium CW-Laser (Wellenlänge von 2,01 µm, Absorptionskoeffizient in Wasser: 64 cm-¹ ergab mit einer 230 µm Faser und einer Laserleistung von 20,5 W an einem Kunststein einen Abtrag von 8,6 mg/min (Totalfragmentation), während bei einer Wellenlänge von 1,92 µm und 25 W ein Abtrag von 45,8 mg/min zu erzielen war, immerhin um einen Faktor 4 höher als bei 2,01 µm. Ein Faktor 2 lässt sich auf den um Faktor 2 geringeren Absorptionskoeffizienten der TM-Wellenlänge von 2,01 µm zurückführen, der verbleibende Faktor 2,5 der verkürzten Impulsdauer zuzuordnen.

Die aufgeführten Messungen beziehen sich auf Dioden gepumpte Faserlaser ohne jede Impulsüberhöhung. Bei Dioden auf Basis (AlGaIN)(GaSb) konnte gezeigt werden, dass Überpulsungen um erhebliche Faktoren erzielt werden können, ohne diese zu zerstören. Damit ergibt sich unter Einsatz multipler Emitter (Barren von z.B. 19 Emittern), Kopplung mehrerer solcher Barren, wie auch bei entsprechend hoher Überpulsung bei Einzelemittern die Möglichkeit, die durch eine Verkürzung der Impulsdauer bedingte Energiereduktion gegebenenfalls vollständig zu kompensieren, mit dem zusätzlichen Effekt, dass die Abtragungseffizienz nochmals ansteigt.

Ähnlich kann in einfacher Weise das Licht von 3 Barren in eine Faser eingekoppelt werden. Unter Verwendung unterschiedlicher Polarisationen kann das Licht von 6 Barren in Fasern eingekoppelt werden und so Energie und Leistung nach oben skaliert werden. Bei entsprechend kurzen Impulsdauern von Pikosekunden können Überpulsbarkeiten um den Faktor 1000 und mehr erwartet werden.

In gleicher Weise können Festkörper- und Faserlaser mit überpulsbaren Dioden gepumpt werden und so die Impulsspitzenleistung gegenüber dem CW-Betrieb deutlich erhöht werden.

## Patentansprüche

1. Multifunktionales Lasergerät zur Bearbeitung von biologischem Material (30), das eine starke Veränderung der Absorptionseigenschaften über den Betriebs-Wellenlängenbereich aufweist, mit mindestens einer aus einer steuerbaren Stromversorgung (10, 20) gespeisten Laserdiode (1), wobei die Stromversorgung mit einer Steuereinrichtung (10) zur Ansteuerung der Laserdiode (1) mit veränderbarem Strom und/oder veränderbarer Impulsdauer und/oder Imputswiederholfrequenz versehen ist,
**dadurch gekennzeichnet, dass** die Steuereinrichtung (10) für eine schnelle Änderung der Laserlicht-Wellenlänge von einer ersten Wellenlänge auf eine zweite Wellenlänge zur Anpassung an den vorgesehenen Anwendungszweck über eine Zeit im Bereich von weniger als einer Sekunde ausgebildet ist, und dass die Steuereinrichtung (10) zur Steuerung des Stromes an die mindestens eine Laserdiode (1) derart ausgebildet ist, dass sich die schnelle Änderung der Wellenlänge über einen Bebiebs-Wellenlängenbereich ergibt, der die Wellenlänge zumindest eines Bereichs mit einer wesentlichen Änderung des Absorptionskoeffizienten des Materials einschließt.

2. Lasergerät nach Anspruch 1, **dadurch gekennzeichnet, dass** die Steuereinrichtung (10) zur Änderung der Wellenlänge durch Änderung des Dauerstrich-Laserdioden-Stromes ausgebildet ist.

3. Lasergerät nach Anspruch 1, **dadurch gekennzeichnet, dass** die Steuereinrichtung (10) zur Änderung der Wellenlänge durch Änderung des Verhältnisses von Impulsdauer zur Impulswiederholzeit des Laserdioden-Stromes ausgebildet ist.

4. Lasergerät nach Anspruch 3, **dadurch gekennzeichnet, dass** die Steuereinrichtung (10) zur Änderung des Verhältnisses von Impulsdauer zur Impulswiederholzeit des Laserdioden-Stromes von Impuls zu Impuls oder von Impulsgruppe zu Impulsgruppe ausgebildet ist.

5. Lasergerät nach Anspruch 3 oder 4, **dadurch gekennzeichnet, dass** die Steuereinrichtung (10) eine erste Betriebsart aufweiset, in der die Impulsdauer während einer Impulswiederholzeit größer als die Impulsdauer für einen Quasi-Dauerstrich-Betrieb ist.

6. Lasergerät nach Anspruch 5, **dadurch gekennzeichnet, dass** die Impulsdauer in der ersten Betriebsart größer als 1µs ist.

7. Lasergerät nach Anspruch 4, **dadurch gekennzeichnet, dass** die Impulsdauer in der ersten Betriebsart größer als 10µs ist.

8. Lasergerät nach einem der Ansprüche 5 bis 7, **dadurch gekennzeichnet, dass** der Diodenlaser-Strom während der Impulse in einer Impulswiederholzeit bei Impulsdauern von 1-100 µs um den Faktor 5 bis 50 gegenüber dem Nennstrom des Diodenlasers vergrößert ist.

9. Lasergerät nach einem der Ansprüche 3 bis 8, **dadurch gekennzeichnet, dass** die Steuereinrichtung eine zweite Betriebsart aufweist, in der die Impulsdauer während einer Impulswiederholzeit kleiner als die Impulsdauer für einen Quasi-Dauerstrich-Betrieb ist.

10. Lasergerät nach Anspruch 9, **dadurch gekennzeichnet, dass** die Impulsdauer in der zweiten Betriebsart im Piko- bis Nanosekunden-Bereich liegt.

11. Lasergerät nach Anspruch 9 oder 10, **dadurch gekennzeichnet, dass** der Diodenlaser-Strom während der Impulse in einer Impulswiederholzeit bei Impulsdauern von 10-1000 ns um den Faktor 10 bis 100 gegenüber dem Nennstrom des Diodenlasers vergrößert ist.

12. Lasergerät nach Anspruche 9 oder 10, **dadurch gekennzeichnet, dass** der Diodenlaser-Strom während der Impulse in einer Impulswiederholzeit bei Impulsdauern von 10ps-10 ns um den Faktor 50 bis1000 gegenüber dem Nennstrom des Diodenlasers vergrößert ist.

13. Lasergerät nach einem der vorhergehenden Ansprüche, **dadurch gekennzeichnet, dass** zumindest zwei Diodenlaser (1) gleicher Wellenlänge vorgesehen sind, deren Ausgangssignal einer Kombinadonseinrichtung zugeführt wird.

14. Lasergerät nach einem der vorhergehenden Ansprüche, **dadurch gekennzeichnet, dass** die Steuereinrichtung (10) zur Veränderung des Stromes, des Verhältnisses von Impulsdauer zur Impulswiederholzeit und der Wellenlänge in Abhängigkeit von dem Ausgangssignal eines Detektionssystems ausgebildet ist, das zur Erkennung von Konkrementen, Blutgefäßen, Nerven, Grenzschichten (Tumor, Prostatakapsel o.a.) über eine Erkennung der Autofluoreszenz von Gewebe, Detektion einer Fluoreszenz nach Anregung über zweite uchtquelle und/oder in Kombination mit OCT ausgebildet ist.

15. Lasergerät nach einem der vorhergehenden Ansprüche, **dadurch gekennzeichnet, dass** die Steuereinrichtung (10) zur Einstellung der Wellenlänge des Ausgangslichts des oder der Diodenlaser (1) auf die Wellenlänge im Bereich der Absorptionsspitze des zu bearbeitenden Materials (30) bei dessen jeweiliger Temperatur ausgebildet ist.

16. Lasergerät nach Anspruch 15, **dadurch gekennzeichnet, dass** die Steuereinrichtung (10) zur Einstellung der Wellenlänge des Ausgangslichts des oder der Diodenlaser (1) auf eine Wellenlänge im Bereich der Absorptionsspitze des zu bearbeitenden Materials (30) ausgebildet ist, bei der die Absorptionsspitze eine geringe Abhängigkeit von der Temperatur des zu bearbeitenden Materials (30) aufweist.

17. Lasergerät nach einem der vorhergehenden Ansprüche, **dadurch gekennzeichnet, dass** das Material biologisches Gewebe ist.

18. Lasergerät nach Anspruch 17, **dadurch gekennzeichnet, dass** der in dem Gewebe enthaltene Absorber Wasser ist.

19. Lasergerät nach Anspruch 17 oder 18, **dadurch gekennzeichnet, dass** die Wellenlänge des Laserlichts im mittleren IR-Bereich von 1,4 bis 3,3 µm liegt.

20. Lasergerät nach Anspruch 19, **dadurch gekennzeichnet, dass** die Wellenlänge des Laserlichts 1,92µm ist.

## Claims

1. Multifunctional laser device for treating material, especially biological material (30), having absorption properties substantially varying over the operating wavelength range, comprising at least one laser diode (1) supplied by a controllable power supply unit (10, 20), the power supply unit having a control device (10) for driving the laser diode (1) with variable current and/or variable pulse duration and/or pulse repetition frequency,
**characterized in that** the control device (10) is adapted for a fast modification of the laser light wavelength from a first wavelength to a second wavelength within a term of less than one second for adaptation to the intended function, and **in that** the control device (10) is adapted for controlling the current supplied to the at least one laser diode (1) such that the fast modification of the wavelength is performed over a range of operating wavelengths which includes the wavelength of at least one range of a substantial variation of the absorption coefficient of the material.

2. Laser device according to claim 1, **characterized in that** the control device (10) is adapted for modifying the wavelength by the varying the continuous wave laser diode current.

3. Laser device according to claim 1, **characterized in that** control device (10) is adapted for modifying the wavelength by varying the ratio of pulse duration to pulse repetition time of the laser diode current.

4. Laser device according to claim 3, **characterized in that** that control device (10) is adapted for modifying the ratio of pulse duration to pulse repetition time of the laser diode current from pulse to pulse or from pulse group to pulse group.

5. Laser device according to claim 3 or 4, **characterized in that** that control device (10) has a first mode of operation wherein the pulse duration during a pulse repetition time is larger then the pulse duration for a quasi continuous wave operation.

6. Laser device according to claim 5, **characterized in that** the pulse duration in the first mode of operation is larger than 1 µs.

7. Laser device according to claim 4, **characterized in that** the pulse duration the first mode of operation is larger than 10µs.

8. Laser device according to any of the claims 5 through 7, **characterized in that** during the pulses of a pulse duration of 1 to 100 µs within a pulse repetition time the laser diode current is increased by a factor of 5 to 50 relative to the rated current of the diode laser.

9. Laser device according to any the claims 3 through 8, **characterized in that** the control device has a second mode of operation wherein the pulse duration within a pulse repetition time is shorter than the pulse duration for a quasi continuous wave operation.

10. Laser device according to claim 9, **characterized in that** the pulse duration in the second mode of operation is within the range of picoseconds to nanoseconds.

11. Laser device according to claim 9 or 10, **characterized in that** during the pulses of a pulse duration of 10 to 1000 ns within a pulse repetition time the laser diode current is increased by a factor of 10 to 100 relative to the rated current of the diode laser.

12. Laser device according to claim 9 or 10, **characterized in that** during the pulses of a pulse duration of 10 ps to 10 ns within a pulse repetition time the laser diode current is increased by a factor of 50 to 1000 relative to the rated current of the diode laser.

13. Laser device according to any of the preceding claims, **characterized in that** at least two diode lasers of the same wavelengths are provided, the output signal thereof being fed to a combination device.

14. Laser device according to any of the preceding claims, **characterized in that** that control device (10) is adapted for varying the current, the ratio of pulse duration to pulse repetition time and the wavelength in response to the output signal of a detection system adapted for detecting concrements, blood vessels, nerves, interfaces (tumor, prostata capsules or others) by detecting the autofluorescence of tissue, detection of a fluorescence on excitation via the second light source and/or in combination with OCT.

15. Laser device according to any of the preceding claims, **characterized in that** the control device (10) is adapted for adjusting the wavelength of the output light of the at least one diode laser (1) to a wavelength in the region of the absorption peak of the material (30) to be treated at the respective temperature thereof.

16. Laser device according to claim 15, **characterized in that** the control device (10) is adapted for adjusting the wavelength of the output light of the at least one diode laser (1) to a wavelength within the region of the absorption peak of the material (30) to be treated at which the absorption peak has a low dependency on the temperature of he material (30) to be treated.

17. Laser device according to any of the preceding claims, **characterized in that** the material is a biological tissue.

18. Laser device according to claim 17, **characterized in that** the absorber contained within the tissue is water.

19. Laser device according to claim 17 or 18, **characterized in that** the wavelength of the laser light is within the medium infrared region of 1.4 to 3.3 µm.

20. Laser device according to claim 19, **characterized in that** the wavelength of the laser light is 1,92 µm.

## Revendications

1. Dispositif laser multifonction pour le traitement d'un matériau biologique (30) qui présente une forte variation des caractéristiques d'absorption sur la gamme de longueurs d'onde de fonctionnement, comportant au moins une diode laser (1) alimentée par une alimentation électrique commandable (10, 20), l'alimentation électrique étant pourvue d'un dispositif de commande (10) permettant de commander la diode laser (1) avec un courant variable et/ou une durée d'impulsion variable et/ou une fréquence de répétition des impulsions variable,
**caractérisé en ce que** le dispositif de commande (10) est conçu pour une variation rapide, de l'ordre de moins d'une seconde, de la longueur d'onde de la lumière laser d'une première longueur d'onde à une deuxième longueur d'onde pour l'adaptation aux applications prévues, et que le dispositif de commande (10) permettant de commander le courant à ladite au moins une diode laser (1) est conçu de façon que la variation rapide de la longueur d'onde se produise sur une gamme de longueurs d'onde de fonctionnement qui inclut la longueur d'onde d'au moins une région présentant une variation substantielle du coefficient d'absorption du matériau.

2. Dispositif laser selon la revendication 1, **caractérisé en ce que** le dispositif de commande (10) est conçu pour une variation rapide de la longueur d'onde par modification du courant de diode laser à émission continue.

3. Dispositif laser selon la revendication 1, **caractérisé en ce que** le dispositif de commande (10) est conçu pour faire varier la longueur d'onde par modification du rapport entre durée d'impulsion et temps de répétition des impulsions du courant de diode laser.

4. Dispositif laser selon la revendication 3, **caractérisé en ce que** le dispositif de commande (10) est conçu pour faire varier le rapport entre durée d'impulsion et temps de répétition des impulsions du courant de diode laser d'une impulsion à une autre ou d'un groupe d'impulsions à un autre.

5. Dispositif laser selon la revendication 3 ou 4, **caractérisé en ce que** le dispositif de commande (10) présente un premier mode de fonctionnement dans lequel la durée d'impulsion pendant un temps de répétition des impulsions est supérieure à la durée d'impulsion pour un fonctionnement à émission quasi continue.

6. Dispositif laser selon la revendication 5, **caractérisé en ce que** la durée d'impulsion dans le premier mode de fonctionnement est supérieure à 1 µs.

7. Dispositif laser selon la revendication 4, **caractérisé en ce que** la durée d'impulsion dans le premier mode de fonctionnement est supérieure à 10 µs.

8. Dispositif laser selon une des revendications 5 à 7, **caractérisé en ce que** pendant les impulsions dans un temps de répétition des impulsions avec des durées d'impulsion de 1 à 100 µs, le courant de diode laser est amplifié d'un facteur 5 à 50 par rapport au courant nominal de la diode laser.

9. Dispositif laser selon une des revendications 3 à 8, **caractérisé en ce que** le dispositif de commande présente un deuxième mode de fonctionnement dans lequel la durée d'impulsion pendant un temps de répétition des impulsions est inférieure à la durée d'impulsion pour un fonctionnement à émission quasi continue.

10. Dispositif laser selon la revendication 9, **caractérisé en ce que** la durée d'impulsion dans le deuxième mode de fonctionnement se situe dans le domaine de la pico- à la nanoseconde.

11. Dispositif laser selon la revendication 9 ou 10, **caractérisé en ce que** pendant les impulsions dans un temps de répétition des impulsions avec des durées d'impulsion de 10 à 1000 ns, le courant de diode laser est amplifié d'un facteur 10 à 100 par rapport au courant nominal de la diode laser.

12. Dispositif laser selon la revendication 9 ou 10, **caractérisé en ce que** pendant les impulsions dans un temps de répétition des impulsions avec des durées d'impulsion de 10 ps à 10 ns, le courant de diode laser est amplifié d'un facteur 50 à 1000 par rapport au courant nominal de la diode laser.

13. Dispositif laser selon une des revendications précédentes, **caractérisé en ce qu'**au moins deux diodes laser (1) de même longueur d'onde sont prévues, dont le signal de sortie est amené à un dispositif de combinaison.

14. Dispositif laser selon une des revendications précédentes, **caractérisé en ce que** le dispositif de commande (10) est conçu pour faire varier le courant, le rapport entre durée d'impulsion et temps de répétition des impulsions et la longueur d'onde en fonction du signal de sortie d'un système de détection qui est conçu pour détecter des concréments, des vaisseaux sanguins, des nerfs, des couches limites (tumeur, capsule prostatique ou analogue) par une détection de l'autofluorescence des tissus, une détection d'une fluorescence après excitation par une deuxième source de lumière et/ou en combinaison avec l'OCT.

15. Dispositif laser selon une des revendications précédentes, **caractérisé en ce que** le dispositif de commande (10) est conçu pour régler la longueur d'onde de la lumière de sortie de la ou des diode(s) laser (1) sur la longueur d'onde dans la région du pic d'absorption du matériau à traiter (30) à sa température respective.

16. Dispositif laser selon la revendication 15, **caractérisé en ce que** le dispositif de commande (10) est conçu pour régler la longueur d'onde de la lumière de sortie de la ou des diode(s) laser (1) sur une longueur d'onde dans la région du pic d'absorption du matériau à traiter (30), à laquelle le pic d'absorption présente une faible dépendance de la température du matériau à traiter (30).

17. Dispositif laser selon une des revendications précédentes, **caractérisé en ce que** le matériau biologique est un tissu.

18. Dispositif laser selon la revendication 17, **caractérisé en ce que** l'absorbeur contenu dans le tissu est de l'eau.

19. Dispositif laser selon la revendication 17 ou 18, **caractérisé en ce que** la longueur d'onde de la lumière laser se situe dans la gamme IR moyenne comprise entre 1,4 et 3,3 µm.

20. Dispositif laser selon 1a revendication 19, **caractérisé en ce que** la longueur d'onde de la lumière laser est de 1,92 µ.m.
